# EUROPEAN PATENT SPECIFICATION

(11) **EP 2 178 500 B1**
(45) Date of publication and mention of the grant of the patent: **04.05.2016**
(21) Application number: 08775998.1
(22) Date of filing: 18.07.2008
(51) Int. Cl.: A61K 9/00, A61K 31/00, A61K 31/31

(54) **DRY-POWDER MEDICAMENT**
TROCKENPULVER-MEDIKAMENT
MÉDICAMENT SOUS FORME DE POUDRE SÈCHE

(30) Priority: 19.07.2007 GB 0714134
(43) Date of publication of application: 28.04.2010
(60) Divisional application: 16153497.9
(73) Proprietor: NORTON HEALTHCARE LIMITED, Castleford West Yorkshire WF10 5HX (GB)
(72) Inventor: ZENG, Xian-Ming, Mitcham, Surrey CR4 2HS (GB); MARTIN, Gary, Peter, Lewes, East Sussex BN7 1LY (GB); MARRIOTT, Christopher, Byfield, Northants NN11 6XN (GB); TAKI, Mohammed, Kingsbury, London NW9 9PN (GB)
(74) Representative: Patent Boutique LLP
(86) International application number: PCT/GB2008/002473
(87) International publication number: WO 2009/010770

(56) References cited:
- WO-A1-03/088944
- SALEEM, I. ET AL: "Modulation of dry powder inhaler performance by controlling micronized drug particle size distributions" AAPS JOURNAL, vol. 8, no. S2, 2006, XP002510592
- BOER, A.H. ET AL.: "Characterization of inhalation aerosols: a critical evaluation of cascade impactor and laser diffraction technique" INT. J. PHARM., vol. 249, 2002, pages 219-231, XP002510593
- FENTON, C. ET AL: "Inhaled salmetrol/fluticasone propionate: a review of its use in chronic obstructive pulmonary disease" DRUGS, vol. 64, no. 17, 2004, pages 1975-1996, XP002510594
- MARKHAM, A. ET AL: "Inhaled salmeterol/fluticasone propionate combination: a pharmacoeconomic review of its use in the management of asthma" PHARMACOECONOMICS, vol. 18, 2000, pages 591-608, XP002510595

## Description

This invention relates to a dry powder medicament and particularly to a medicament which provides improved inhalation properties.

Inhalable medicaments are often formulated as dry powders and are delivered using a dry-powder inhaler (DPI). They are inhaled through the mouth-and into the lungs. The drug is either preloaded into the inhaler, or filled into capsules, e.g. gelatine capsules, or blister packs. Examples of DPIs may be found in "Pharmaceutics - The science of dose form design" Second Edition, Ed. M .E. Aulton, Churchill Livingston, 2002, chapter 31.

The medicaments are typically those used to treat respiratory diseases such as asthma and COPD. Examples of suitable medicaments include anti-allergic agents (e.g. cromoglycate, ketotifen and nedocromil), anti-inflammatory steroids (e.g. beclomethasone dipropionate, fluticasone, budesonide, flunisolide, ciclesonide, triamcinolone acetonide and mometasone furoate); bronchodilators such as: β₂-agonists (e.g. fenoterol, formoterol, pirbuterol, reproterol, salbutamol, salmeterol and terbutaline), non-selective β-stimulants (e.g. isoprenaline), and xanthine bronchodilators (e.g. theophylline, aminophylline and choline theophyllinate); and anticholinergic agents (e.g. ipratropium bromide, oxitropium bromide and tiotropium).

The medicaments are micronised using conventional techniques (e.g. a jet mill) to produce a suitable particle size for inhalation which is typically in the region of 5 µm or less in diameter. The high-energy particles produced by micronisation tend to have poor flow properties on account of their static, cohesive and adhesive nature. To improve the flow properties, the medicament is blended with larger carrier particles of an inert excipient, usually lactose. The coarse carrier usually has a particle size which is an order of magnitude larger than the micronised medicament, typically around 20-100 µm in diameter. A preferred carrier is coarse lactose, preferably having a particle size of 63-90 µm in diameter.

On inhalation, the turbulent air flow generated in the inhaler causes deaggregation of the carrier particles and the drug particles. The larger carrier particles impact on the throat whereas a proportion of the smaller drug particles are entrained into the respiratory tract. However, a balance has to struck between adhesion of the drug particles to the carrier in order to provide the appropriate flow properties, and the subsequent desorption of the drug from the carrier on inhalation. The formulator must consider all of the factors including the chemical and physical (particle size, shape, density, surface roughness, hardness, moisture content, bulk density etc) properties of both the drug and carrier. The interaction between the drug and the carrier is further complicated where the drug is presented as a combination product including two or more different active ingredients. In addition, inhalable medicaments often employ fine inert particles, such as fine lactose, in order to assist in the deaggregation of the drug and the carrier. It is thought that the fine carrier takes up binding sites on the surface of the carrier resulting in a weaker interaction between the drug and the coarse carrier which facilitates deaggregation.

Several approaches have been proposed to improve the delivery of the medicament. For example, improvements to the inhaler have been proposed to assist in deagglomeration of the medicament. See US 6,871,646, US 6,748,947 and US 5,503,144 by way of examples. The particles themselves may also be modified, for example to improve the shape or surface smoothness. Methods of precise mixing of the dry powder preparations have also been proposed improve dose uniformity, reliability and dispersion, see WO 2004/017918.

However, there remains a need in the art for techniques which improve further the physical properties of the dry-powder medicament, and particularly which improve the FPF.

Saleem, I. et al: "Modulation of dry powder inhaler performance by controlling micronized drug particle size distributions", AAPS JOURNAL, vol. 8, no. S2, 2006 is a study into the influence of tightly controlled micronised drug particle size distributions on the in vitro performance of a model dry powder formulation. In Boer, A.H. et al.: "Characterization of inhalation aerosols: a critical evaluation of cascade impactor and laser diffraction technique", Int. J. Pharm., vol. 249, 2002, pages 219-231, the applicability of laser diffraction technology is evaluated as an alternative for cascade impactor analysis. FENTON, C. ET AL: "Inhaled salmeterol/fluticasone propionate: a review of its use in chronic obstructive pulmonary disease", DRUGS, vol. 64, no. 17, 2004, pages 1975-1996 and Markham, A. et al: "Inhaled salmeterol/fluticasone propionate combination: a pharmacoeconomic review of its use in the management of asthma", Pharmacoeconomics, vol. 18, 2000, pages 591-608 investigate the combined use of salmeterol and fluticasone propionate. WO 03/088944 relates to dry powder pharmaceutical compositions comprising a bronchodilator drug in combination with a steroidal anti-inflammatory drug, dry powder inhalers comprising the same and their use in the treatment of respiratory disorders by inhalation.

Accordingly, the present invention provides a method for preparing an inhalable dry-powder medicament comprising the steps of: (i) fractionating a particulate active ingredient based on aerodynamic particle size, (ii) recovering at least one fraction of the particulate active ingredient and (iii) combining the recovered fraction with a carrier to provide the inhalable dry-powder medicament; further comprising the steps of fractionating one or more further particulate ingredients based on aerodynamic particle size, recovering at least one fraction of the one or more further particulate ingredients and combining the recovered fraction(s) with the inhalable dry-powder medicament; wherein the one or more further particulate ingredients comprise further particulate active ingredient and a particulate fine carrier; wherein the particulate fine carrier is fine lactose; and wherein the particulate active ingredient and further particulate active ingredient comprise fluticasone propionate and salmeterol xinafoate.

The present invention also provides an inhalable dry-powder medicament obtainable by the above method; as well as an inhalable dry-powder medicament comprising a particulate active ingredient and a carrier, wherein the particulate active ingredient is pre-fractionated based on aerodynamic particle size; further comprising one or more further particulate ingredients which are pre-fractionated based on aerodynamic particle size; wherein the one or more further particulate ingredients comprise a further particulate active ingredient and a particulate fine carrier; wherein the particulate fine carrier is fine lactose; and wherein the particulate active ingredient and further particulate active ingredient comprise fluticasone propionate and salmeterol xinafoate.

Thus, the present applicant has found that an improved medicament may be produced by fractionating the medicament prior to formulation. Fractionation was previously a technique only considered for analytical purposes.

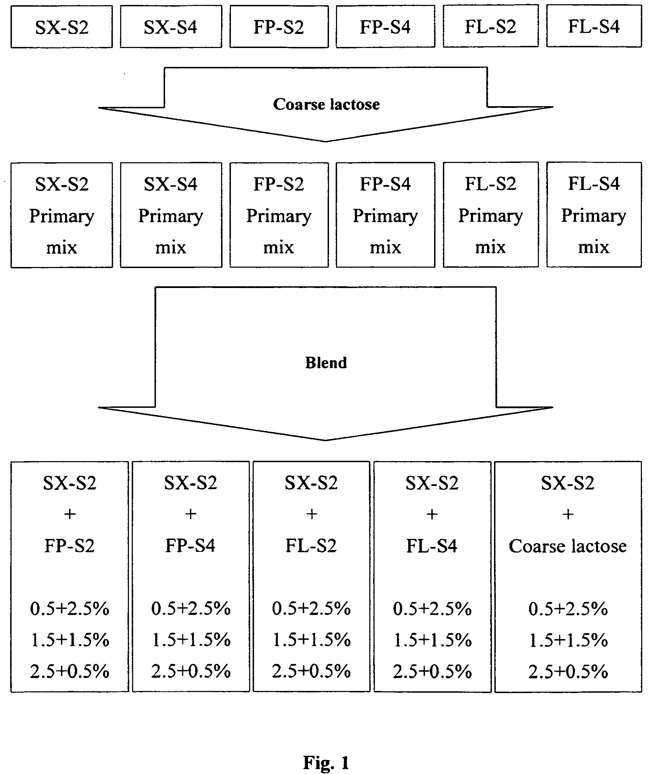

The present invention will now be described with reference to the following drawing, in which Fig. 1 represents a schematic diagram of the preparation of the samples set out in Tables 4-15.

The method of the present invention involves the provision of a particulate active ingredient which will typically have been micronised using standard techniques. The medicament is then fractionated based on aerodynamic particle size and a limited number of the collected fractions are used to form the dry-powder medicament.

Fractionation is achieved by entraining the particulate medicament in an airstream in a suitable apparatus. The aerosol is passed though progressively finer jets and collecting plates. The particles impact on the collection plates and may be collected. Fraction of medical aerosols is a well known technique, but to-date the techniques have only been used for particle size analysis.

Several methods have been developed for the analysis of particle size. The distribution of particles in vivo can be measured using techniques such as radiolabelling. However, more commonly, in vitro methods are employed to measure particle size with a view to predicting the effects in patients. One such technique involves fractionation using cascade impactors and impingers.

Cascade impactors comprise a series of progressively finer jets and collection plates allowing fractionation of aerosols according to their mass median aerodynamic particle size (MMAD) as the particles are drawn through the impactor at a known flow rate. The collection plates are treated with an adhesive to hold the particles. Multistage liquid impingers work on a similar principle but tend to have wet glass collection plates. Both are listed in the USP and Ph. Eur. for the analysing particle size in medical aerosols.

The fractionation used in the present invention is preferably performed using a Next Generation Pharmaceutical Impinger (NGI). See V.A. Marple et al "Next Generation Pharmaceutical Impactor. Part 1: Design" J. Aerosol Med. 2003; 16:283-299 and V.A. Marple et al "Next Generation Pharmaceutical Impactor. Part 2: Archival calibration" J. Aerosol Med. 2003; 16:301-324 for further details. The NGI stage cut-off diameters at a flow rate of 60 L/min are described in the Ph. Eur. as shown in Table 1.

Table 1. NGI stage cut-off diameters.

| **Stage** | **Upper limit (µm)** | **Lower limit (µm)** |
|---|---|---|
| S1 | >8.06 | 8.06 |
| S2 | 8.06 | 4.46 |
| S3 | 4.46 | 2.82 |
| S4 | 2.82 | 1.66 |
| S5 | 1.66 | 0.94 |
| S6 | 0.94 | 0.55 |
| S7 | 0.55 | 0.34 |
| S8 | 0.34 | - |

The fractions used in the dry-powder medicament of the present invention will depend on the nature of the active ingredient. However, the desired fraction or fractions may be recovered and at least one fraction of the particulate active ingredient is employed as the dry powder. The recovered fraction or fractions is preferably taken from a stage or stages having an upper cut-off limit of 7.0-9.0 µm and a lower cut-off limit of 2.5-3.0 µm determined at a flow rate of 60 ± 5 L/min. More preferably the lower cut-off limit is 4.0-6.0 determined at a flow rate of 60 ± 5 L/min. More preferably the upper cut-off limit is 8.0 µm and the lower cut-off limit is 2.8 µm, more preferably the lower cut-off limit is 4.4 µm determined at a flow rate of 60 ± 5 L/min.

The particles in the recovered fraction of the particulate active ingredient preferably have an aerodynamic particle size of 1.0 to 5.0 µm, more preferably 1.5 to 4.5 µm and most preferably 2.0 to 3.5 µm. The aerodynamic particle size may be measured according to the Ph. Eur. method at a flow rate of 60 ± 5 L/min. Since the particles have been fractionated, substantially all of the particles will have an aerodynamic particle size falling within the afore-mentioned ranges. By "substantially all" is meant that the particles have been fractionated such that they fall within this range, but within the limits inherent in method of fractionation used. Typically, 90% of the particles will fall within this range, more preferably 95%.

The recovered fraction is then combined with a carrier, such as coarse lactose, to provide the inhalable dry-powder medicament.

The method further comprises the steps of fractionating one or more further particulate ingredients based on aerodynamic particle size, recovering at least one fraction of the one or more further particulate ingredients and combining the recovered fraction(s) with the inhalable dry-powder medicament. The one or more further particulate ingredients comprise further particulate active ingredient and a particulate fine lactose carrier. The one or more further particulate ingredients preferably have an aerodynamic particle size of 1.0 to 5.0 µm, more preferably 1.5 to 4.5 µm and most preferably 2.0 to 3.5 µm.

The particulate active ingredient and further particulate active ingredient comprise fluticasone propionate and salmeterol xinafoate.

The present invention also provides a capsule comprising the medicament described herein and a dry-powder inhaler comprising the capsule or the medicament described herein.

By way of an example, the present invention will now be described with reference to the active ingredients salmeterol xinafoate (SX) and fluticasone propionate (FP).

Micronised samples of salmeterol xinafoate (SX) obtained from Vamsi Labs Ltd, Maharashtra, India; fluticasone propionate (FP) obtained from Coral Drugs Ltd, New Delhi, India; and fine lactose (FL) obtained from Friesland Foods Domo, Zwolle, The Netherlands, were aerosolised into an NGI (MSP Corporation, Shoreview, MN, USA) by a Dry Powder Feeder (Malvern Instruments Ltd, Worcestershire, UK) at a flow rate of 50 L/min and a slow powder feed rate was chosen (mark 2).

The feeder employs two opposing air jets to separate particles and break up agglomerates. The NGI was connected to a vacuum pump and the flow was set to 60 L/min. The powder exit port of the Dry Powder Feeder was aligned with the USP throat of the NGI allowing the powder to flow directly into the impactor.

A pre-separator was used to separate coarse particles and agglomerates but no liquid was placed in the pre-separator to allow the recovery of the solid particles after the run. Similarly, no coating was applied to the collection cups of the NGI to allow the recovery and reuse of the deposited powder.

The experiment was run for 1 min, the powder feed was then stopped and the vacuum pump was switched off. The deposits were then recovered from each NGI stage and the nozzles of the NGI stages checked to ensure they were blockage-free. The experiment was then resumed for further periods of 1 min until sufficient quantities of powder was recovered.

The recovered powders were then transferred into glass vials and stored in a desiccator containing silica gel until required.

The NGI stage cut-off diameters at 60 L/min were calculated as described in the European Pharmacopoeia and shown in Table 1 hereinabove.

The particle sizes of the salmeterol xinafoate (SX) and the fluticasone propionate (FP) thus obtained are set out in Tables 2 and 3.

**Table 2. Fluticasone Propionate ("FP"), Malvern, n=6**

| **Stage** | **D_{0.1}** | **D_{0.5}** | **D_{0.9}** | **Span** |
|---|---|---|---|---|
| **S1** | 1.01 | 2.56 | 6.06 | 1.77 |
| **S2** | 1.00 | 2.49 | 5.28 | 1.72 |
| **S3** | 0.95 | 2.20 | 4.51 | 1.62 |
| **S4** | 0.86 | 1.89 | 3.72 | 1.52 |
| **S5** | 0.73 | 1.61 | 3.22 | 1.55 |
| **S6** | 0.65 | 1.28 | 2.95 | 1.79 |
| **S7** | - | - | - | - |
| **S8** | - | - | - | - |
| **Micronised FP** | 1.23 | 3.57 | 7.43 | 1.74 |

**Table 3. Salmeterol Xinafoate ("SX"), Malvern, n=6**

| **Stage** | **D_{0.1}** | **D_{0.5}** | **D_{0.9}** | **Span** |
|---|---|---|---|---|
| **S1** | 0.69 | 1.99 | 6.08 | 2.70 |
| **S2** | 0.69 | 1.87 | 5.73 | 2.69 |
| **S3** | 0.68 | 1.78 | 4.61 | 2.23 |
| **S4** | 0.66 | 1.54 | 3.93 | 2.21 |
| **S5** | 0.64 | 1.36 | 3.47 | 2.08 |
| **S6** | 0.63 | 1.17 | 3.23 | 2.23 |
| **S7** | 0.60 | 1.10 | 3.11 | 2.28 |
| **S8** | 0.58 | 0.97 | 2.19 | 1.65 |
| **Micronised SX** | 0.74 | 2.20 | 5.09 | 1.98 |

The precise values for the diameter (D_{0.1}, D_{0.5} and D_{0.9}) are different for the two active ingredients because the different densities will lead to different aerodynamic particle sizes. Stages 2 and 4 have been highlighted as these fractions are used in the examples set out hereinbelow.

Coarse lactose was obtained from and dry sieved to obtain a fraction between 63 and 90 µm as typically used in DPI formulations. The sample was sieved three times to minimise the number of particles outside the required range.

Fine drug and lactose samples recovered from stages 2 and 4 of the NGI were chosen for further experimentation as they both have MMAD values between 1 and 5 µm and are therefore considered suitable for deep-lung deposition. They are also broadly in the ranges used in DPI formulations available commercially.

The mixing sequence is shown schematically in Fig. 1. Each sample was geometrically mixed with coarse lactose in a 1:15 (w/w) ratio to prepare a primary mix. The mixing process involved weighing equal amounts of drug and coarse lactose into a 20 mL glass vial. The samples where then mixed using a Whirlimixer (Fisons Scientific Equipment, Leicester, UK) for 1 min. An equivalent weight of coarse lactose was then added to the contents of the vial and the samples mixed for 1 min as described (four stages in total). Once the required amount of coarse lactose had been added, the contents were tumble-mixed in a Turbula mixer (Messrs Bachofen AG, Basel, Switzerland) for 30 min.

The homogeneity of the mix was validated by accurately weighing 10 samples each containing 2-3 mg and dissolving them in 10 mL using volumetrics. The contents were then quantified using the validated HPLC method. The RSD for all mixes was <2%.

The HPLC instrument used in the validated HPLC method was a SpectraPHYSICS (trade mark) system (Thermoseparation Products Inc., California, USA) containing a pump (P1000), an auto-sampler (AS1000) and a UV detector (UV1000). The column used was a ThermoQuest (Cheshire, UK) hypersil column (C18, 4.6 mm, 5 µm, 25 cm). The mobile phase was a mixture of methanol and 0.2% ammonium acetate buffer at pH 4 (± 0.01) in a ratio of 75:25, respectively. The flow rate was 1.00 mL/min and the temperature was 40°C. Detection was made using a UV detector set at a wavelength of 228 nm.

The primary mix was used to obtain all samples in the next stages of the mix to ensure their equivalence.

The total percentage of fine particles added to each formulation was kept constant at 3% to avoid variability resulting from changes in the drug to carrier ratio. Therefore, samples contained either: 0.5% A and 2.5% B, 1.5% of each, or 2.5% A and 0.5% B, where A and B are either SX-S2, SX-S4, FP-S2, FP-S4, FL-S2 or FL-S4. Samples were also made where B was coarse lactose to investigate the effect of fine particles - regardless of their nature - in the formulation.

Appropriate amounts (depending on the ratio) were then obtained from the primary mixes and mixed for 1 min using a Whirlimixer. Coarse lactose was then geometrically added as described above. Mixing validation was carried out as set out hereinabove and RSD values for all mixes were <2%.

For each of the final mixes (containing 0.5, 1.5 or 2.5% of drug), 20 hard gelatin capsules (size 3, Meadow Laboratories Ltd, Romford, Essex, UK) were prepared each containing 30 mg (± 1 mg) and were used for the deposition studies.

The device used in the deposition studies was an Aerolizer (Novartis Pharma, Basel, Switzerland) and the impactor used was an NGI at a flow rate of 60 L/min. Collection cups were coated and samples recovered using the HPLC method above.

Each capsule was placed into the Aerolizer and pierced as prescribed. The device was then attached to the USP throat of the NGI by means of a suitable rubber adapter providing a seal. The vacuum pump attached to the NGI was then started for 4 seconds allowing 4 L to pass through the device. A total of 5 capsules (150 mg) were actuated per run. The mass median aerodynamic diameter (MMAD), geometric standard deviation (GSD) and fine particle fraction (FPF) at less than 3 and 5 µm were measured. The MMAD and GSD provide the central tendency and spread, respectively and the FPF of the medicament is a measure of the proportion of particles below a given particle size compared to the total mass emitted by the inhaler. Techniques are available in the art to achieve a large FPF, but principally micronisation of the drug is employed.

The results are set out in the following Tables 4-18.

**Table 4. SX-S2,1.5% + Coarse Lactose, 1.5%.**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 1.51 | | 1.88 | | 20.44 | | 23.04 | |
| Run 2 | 1.42 | | 1.87 | | 23.96 | | 26.50 | |
| Run 3 | 1.47 | | 1.83 | | 24.17 | | 26.84 | |
| Run 4 | 1.47 | | 1.80 | | 23.55 | | 26.00 | |
| Mean (n=4) | 1.46 | | 1.85 | | 23.03 | | 25.60 | |
| % RSD | 2.6 | | 2.1 | | 7.6 | | 6.8 | |

**Table 5. SX-S2, 1.5% + FL-S2, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 1.53 | | 1.86 | | 25.92 | | 29.28 | |
| Run 2 | 1.41 | | 1.94 | | 28.77 | | 32.06 | |
| Run 3 | 1.44 | | 1.85 | | 28.73 | | 31.83 | |
| Run 4 | 1.45 | | 1.86 | | 27.41 | | 30.44 | |
| Mean (n=4) | 1.46 | | 1.88 | | 27.71 | | 30.90 | |
| % RSD | 3.5 | | 2.3 | | 4.9 | | 4.2 | |

**Table 6. SX-S2,1.5% + FL-S4, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FPF | SX | FP | SX | FP |
| Run 1 | 1.47 | | 1.86 | | 20.56 | | 22.95 | |
| Run 2 | 1.41 | | 1.91 | | 22.95 | | 25.48 | |
| Run 3 | 1.43 | | 1.90 | | 23.35 | | 26.00 | |
| Run 4 | 1.48 | | 1.86 | | 21.33 | | 23.84 | |
| Mean (n=4) | 1.45 | | 1.88 | | 22.05 | | 24.57 | |
| % RSD | 2.2 | | 1.4 | | 6.0 | | 5.8 | |

**Table 7. SX-S2,1.5% + FP-S2, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 1.44 | 1.54 | 1.83 | 1.88 | 25.64 | 19.47 | 28.31 | 22.06 |
| Run 2 | 1.49 | 1.57 | 1.74 | 1.78 | 23.43 | 18.31 | 25.75 | 20.59 |
| Run 3 | 1.43 | 1.52 | 1.80 | 1.85 | 25.03 | 18.91 | 27.47 | 21.27 |
| Run 4 | 1.51 | 1.59 | 1.74 | 1.78 | 22.56 | 17.12 | 24.87 | 19.33 |
| Mean (n=4) | 1.46 | 1.55 | 1.78 | 1.82 | 24.16 | 18.45 | 26.60 | 20.81 |
| % RSD | 2.7 | 1.9 | 2.6 | 2.8 | 5.9 | 5.4 | 5.9 | 5.6 |

**Table 8. SX-S2,1.5%+ FP-S4, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 1.39 | 1.35 | 1.87 | 1.94 | 25.83 | 18.18 | 28.44 | 20.05 |
| Run 2 | 1.48 | 1.44 | 1.78 | 1.84 | 25.16 | 17.92 | 27.84 | 19.83 |
| Run 3 | 1.46 | 1.41 | 1.78 | 1.84 | 27.30 | 19.55 | 30.05 | 21.49 |
| Run 4 | 1.43 | 1.39 | 1.85 | 1.89 | 25.62 | 18.17 | 28.28 | 20.05 |
| Mean (n=4) | 1.44 | 1.40 | 1.82 | 1.88 | 25.98 | 18.45 | 28.65 | 20.35 |
| % RSD | 2.7 | 2.6 | 2.4 | 2.6 | 3.6 | 4.0 | 3.4 | 3.8 |

**Table 9. SX-S4,1.5% + FP-S2, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 1.54 | 1.77 | 1.83 | 1.84 | 14.27 | 10.36 | 16.06 | 12.28 |
| Run 2 | 1.60 | 1.84 | 1.77 | 1.78 | 12.90 | 9.22 | 14.59 | 11.02 |
| Run 3 | 1.49 | 1.70 | 1.83 | 1.88 | 14.11 | 10.47 | 15.74 | 12.28 |
| Run 4 | 1.59 | 1.84 | 1.85 | 1.87 | 11.84 | 9.15 | 13.51 | 11.04 |
| Mean (n=4) | 1.55 | 1.79 | 1.82 | 1.84 | 13.28 | 9.80 | 14.97 | 11.66 |
| % RSD | 3.4 | 3.6 | 1.9 | 2.4 | 8.6 | 7.3 | 7.8 | 6.2 |

**Table 10. SX-S4,1.5% + FP-S4, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 1.51 | 1.63 | 1.77 | 1.80 | 14.25 | 9.40 | 15.83 | 10.73 |
| Run 2 | 1.51 | 1.60 | 1.82 | 1.87 | 13.58 | 8.51 | 15.19 | 9.75 |
| Run 3 | 1.55 | 1.65 | 1.79 | 1.82 | 12.83 | 8.51 | 14.40 | 9.80 |
| Run 4 | 1.52 | 1.62 | 1.81 | 1.85 | 14.29 | 9.92 | 15.98 | 11.40 |
| Mean (n=4) | 1.53 | 1.63 | 1.80 | 1.83 | 13.74 | 9.09 | 15.35 | 10.42 |
| % RSD | 1.2 | 1.3 | 1.2 | 1.7 | 5.0 | 7.7 | 4.7 | 7.6 |

**Table 11. SX-S4,1.5% + FL-S2, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 1.44 | | 1.90 | 22.59 | 22.59 | | 25.18 | |
| Run 2 | 1.49 | | 1.92 | | 21.97 | | 24.82 | |
| Run 3 | 1.53 | | 1.95 | | 21.85 | | 24.91 | |
| Run 4 | 1.50 | | 1.93 | 21.45 | 21.45 | 24.30 | 24.30 | |
| Mean (n=4) | 1.49 | | 1.93 | 21.96 | 21.96 | 24.80 | 24.80 | |
| % RSD | 2.6 | | 1.0 | | 2.2 | | 1.5 | |

**Table 12. SX-S4,1.5% + FL-S4, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 1.48 | | 1.91 | | 18.81 | 21.14 | 21.14 | |
| Run 2 | 1.52 | | 1.87 | | 16.51 | | 18.61 | |
| Run 3 | 1.47 | | 1.93 | | 15.90 | | 17.88 | |
| Run 4 | 1.49 | | 1.91 | | 16.62 | | 18.74 | |
| Mean (n=4) | 1.49 | | 1.90 | | 16.96 | | 19.09 | |
| % RSD | 1.5 | | 1.4 | | 7.5 | | 7.4 | |

**Table 13. FP-S2,1.5% + FL-S4, 1.5%**

| **MMAD (µm)** | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | | 1.84 | | 1.82 | | 12.25 | | 14.73 |
| Run 2 | | 1.77 | | 1.93 | | 12.25 | | 14.65 |
| Run 3 | | 1.73 | | 1.89 | | 11.38 | | 13.45 |
| Run 4 | | 1.92 | | 1.89 | | 10.25 | | 12.62 |
| Mean (n=4) | | 1.82 | | 1.88 | | 11.53 | | 13.86 |
| % RSD | | 4.5 | | 2.4 | | 8.2 | | 7.3 |

**Table 14. FP-S4, 1.5% + FL-S4, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | | 1.79 | | 1.92 | | 6.56 | | 7.87 |
| Run 2 | | 1.84 | | 1.92 | | 6.09 | 7.39 | 7.39 |
| Run 3 | | 1.76 | | 1.88 | | 7.45 | | 8.86 |
| Run 4 | | 1.80 | | 1.92 | | 6.62 | | 7.97 |
| Mean (n=4) | | 1.80 | | 1.91 | | 6.68 | | 8.02 |
| % RSD | | 1.8 | | 1.1 | | 8.5 | | 7.6 |

**Table 15. FP-S4,1.5% + FL-S2, 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | | 1.82 | | 1.92 | | 10.28 | | 12.40 |
| Run 2 | | 1.80 | | 1.95 | | 10.57 | | 12.74 |
| Run 3 | | 1.91 | | 2.06 | | 10.38 | | 12.85 |
| Run 4 | | 1.80 | | 2.05 | | 10.69 | | 12.95 |
| Mean (n=4) | | 1.83 | | 1.99 | | 10.48 | | 12.74 |
| % RSD | | 2.9 | | 3.6 | | 1.8 | | 1.9 |

**Table 16. SX-UF1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | 2.37 | | 2.39 | | 6.29 | | 8.33 | |
| Run 2 | 2.38 | | 2.32 | | 6.65 | | 8.87 | |
| Run 3 | 2.44 | | 2.33 | | 7.12 | | 9.56 | |
| Run 4 | 2.38 | | 2.20 | | 7.58 | | 10.18 | |
| Mean (n=4) | 2.39 | | 2.31 | | 6.91 | | 9.24 | |
| % RSD | 1.3 | | 3.5 | | 8.1 | | 8.7 | |

**Table 17. FP-UF 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | | 2.76 | | 1.81 | | 2.84 | | 4.30 |
| Run 2 | | 2.72 | | 1.90 | | 3.26 | | 4.83 |
| Run 3 | | 2.87 | | 1.76 | | 2.62 | | 4.13 |
| Run 4 | | 2.90 | | 1.75 | | 2.72 | | 4.33 |
| Mean (n=4) | | 2.81 | | 1.80 | | 2.86 | | 4.40 |
| % RSD | | 2.9 | | 3.8 | | 9.9 | | 6.8 |

**Table 18. FP-S2 1.5%**

| | **MMAD (µm)** | | **GSD** | | **FPF_{<3 µm}** | | **FPF_{<5 µm}** | |
|---|---|---|---|---|---|---|---|---|
| Active | SX | FP | SX | FP | SX | FP | SX | FP |
| Run 1 | | 2.61 | | 1.61 | | 4.35 | | 6.46 |
| Run 2 | | 2.42 | | 1.64 | | 4.62 | | 6.42 |
| Run 3 | | 2.34 | | 1.70 | | 5.70 | | 7.74 |
| Run 4 | | 2.50 | | 1.66 | | 5.27 | | 7.52 |
| Mean (n=4) | | 2.47 | | 1.65 | | 4.98 | | 7.03 |
| % RSD | | 4.6 | | 2.3 | | 12.3 | | 9.8 |

Table 19 provides a summary of the components used in the experiments set out in Tables 4-18 as well as the mean FPF_{<5 µm} which is shown in parentheses.

**Table 19. Summary of mean FPF_{<5 µm}**

| **Table** | **Composition (FPF_{<5 µm})** |
|---|---|
| T4 | SX-S2 (25.60) |
| T5 | SX-S2 + FL-S2 (30.90) |
| T6 | SX-S2 + FL-S4 (24.57) (20.81) |
| T7 | SX-S2 + FP-S2 (28.65 (20.81)) |
| T8 | SX-S2 + FP-S4 (28.65) (20.35) |
| T9 | SX-S4 + FP-S2 (14.97) (10.42) |
| T10 | SX-S4 + FP-S4 (15.35) (10.42) |
| T11 | SX-S4 + FL-S2 (24.57) |
| T12 | SX-S4 + FL-S4 (24.80) |
| T13 | FP-S2 + FL-S4 (13.86) |
| T14 | FP-S4 + FL-S4 (8.02) |
| T15 | FP-S4 + FL-S2 (12.74) |
| T16 | SX-UF (9.24) |
| T17 | FP-UF (4.40) |
| T18 | FP-S2 (7.03) |

Table 4 is provided as a control experiment in that salmeterol xinafoate (SX-S2) is tested alone, i.e. only in the presence of coarse lactose but no other fine particles. The mean FPF_{<5 µm} is 25.60. Table 5 shows the combination of SX-S2 and fine lactose (FL-S2) produces a higher FPF_{<5 µm} of 30.90. A similar increase in the FPF_{<3 µm} may also be seen. This result is expected since it is known in the art that combining fine lactose increases the FPF of the drug itself. Without wishing to be bound by theory, it is understood that the fine lactose adheres to the surface of the coarse lactose reducing the adhesion of the drug, thereby allowing more of the drug to become free of the carrier on inhalation.

One would therefore expect that a finer grade of lactose (a lower MMAD) would provide still further improvements in the FPF. However, Table 6 shows the combination of the same grade of salmeterol xinafoate (SX-S2), but with finer lactose (FL-S4) - the higher fraction of the impinger is a smaller particle size - results in a reduced FPF_{<5 µm} of 24.57. The present applicant has therefore surprisingly found that limiting the minimum, as well as the maximum, particle size improves the FPF.

The same observation may be made for fluticasone propionate (FP-S4) by comparing Tables 14 and 15 which shows that decreasing the particle size of the fine lactose (FL-S2 to FL-S4) decreases the FPF_{<5 µm} of the fluticasone propionate (from 12.74 to 8.02).

This effect may be seen in greater contract by comparing micronised but unfractionated salmeterol xinafoate (SX-UF) and micronised but unfractionated fluticasone propionate (FP-UF), with fractionated salmeterol xinafoate (SX-S2) and fractionated fluticasone propionate (FP-S2). Table 16 shows that the FPF_{<5 µm} of SX-UF is 9.24 whereas Table 3 shows that the FPF_{<5 µm} of SX-S2 is 25.60. Similarly, comparing Tables 17 and 18 shows that the FPF_{<5 µm} of FP-UF is 4.40 whereas the FPF_{<5 µm} of FP-S2 is 7.03.

In addition, the nature of the drugs when used in combination can also have an effect on the FPF. Comparing Tables 4 and 7 shows that the deposition of SX-S2 is essentially the same in the presence or absence of FP-S2 (FPF_{<5 µm} of 25.60 compared to 26.60).

However, when these two drugs are combined, it is preferable to use SX-S2 rather than SX-S4. In this regard, comparing Tables 7 and 9 shows that the FPF_{<5 µm} for SX-S2 in the presence of FP-S2 is 26.60, but drops to 14.97 when SX-S4 is used in the presence of the same FP-S2. Similarly, comparing Tables 8 and 10 shows that SX-S2 is also preferable to SX-S4 in the presence of FP-S4. These are surprising results since one would expect that the smaller particles sizes (S4 over S2) would result in a higher FPF.

The same trend may be seen for salmeterol xinafoate in the presence of fine lactose, cf. Tables 5 and 11, and Tables 6 and 12 which compare SX-S2 and SX-S4 in the presence of FL-S2 and FL-S4, respectively.

Similarly, the trend can also be seen for fluticasone propionate. Comparing Tables 13 and 14 shows that the fractions S2 and S4 obtained from the different stages of the NGI are different. Again, the FPF_{<5 µm} for FP-S2 is higher than for the smaller particle size grade FP-S4 in the presence of the same grade of fine lactose (FL-S4).

Comparing samples Table 5 and 7 as well as Tables 6 and 8 shows that the physicochemical properties of co-drug/fine particles is also important. The FPF_{<5 µm} for SX-S2 and SX-S4 decreases when fluticasone propionate is used in place of fine lactose. In addition, comparing Tables 7 and 8, and Tables 9 and 10 shows that the particle size of fluticasone propionate has minimal affect on the deposition of salmeterol xinafoate.

It is thought that the reason that the size of salmeterol xinafoate influences the FPF of fluticasone propionate but not the reverse, is due to salmeterol xinafoate being the less cohesive drug. Salmeterol xinafoate, in this case, is less agglomerated and would not be expected to benefit from the more cohesive large fluticasone propionate agglomerates. On the other hand, any interaction between the drugs allows salmeterol xinafoate to penetrate the fluticasone propionate clusters producing smaller, more open-packed agglomerates.

Thus, the present invention also provides a medicament comprising salmeterol xinafoate and fluticasone propionate having particles sizes as defined herein. More preferably, the salmeterol xinafoate and fluticasone propionate have an aerodynamic particle size from 1.0 to 5.0 µm, and most preferably from 2.0 to 3.5 µm.

## Claims

1. A method for preparing an inhalable dry-powder medicament comprising the steps of:
(i) fractionating a particulate active ingredient based on aerodynamic particle size,
(ii) recovering at least one fraction of the particulate active ingredient and
(iii) combining the recovered fraction with a carrier to provide the inhalable dry-powder medicament;
further comprising the steps of fractionating one or more further particulate ingredients based on aerodynamic particle size, recovering at least one fraction of the one or more further particulate ingredients and combining the recovered fraction(s) with the inhalable dry-powder medicament;
wherein the one or more further particulate ingredients comprise further particulate active ingredient and a particulate fine carrier;
wherein the particulate fine carrier is fine lactose; and
wherein the particulate active ingredient and further particulate active ingredient comprise fluticasone propionate and salmeterol xinafoate.

2. A method as claimed in claim 1, wherein the fractionation is performed using an NGI.

3. A method as claimed in claim 2, wherein the recovered fraction is from a stage or stages having an upper cut-off limit of 7.0-9.0 µm and a lower cut-off limit of 2.5-3.0 µm determined at a flow rate of 60±5 L/min.

4. An inhalable dry-powder medicament comprising a particulate active ingredient and a carrier, wherein the particulate active ingredient is pre-fractionated based on aerodynamic particle size;
further comprising one or more further particulate ingredients which are pre-fractionated based on aerodynamic particle size;
wherein the one or more further particulate ingredients comprise a further particulate active ingredient and a particulate fine carrier;
wherein the particulate fine carrier is fine lactose; and
wherein the particulate active ingredient and further particulate active ingredient comprise fluticasone propionate and salmeterol xinafoate.

5. A method as claimed in any preceding claim, wherein the carrier is coarse lactose.

6. A method as claimed in any preceding claim, wherein the particles in the recovered fraction of the particulate active ingredient have an aerodynamic particle size of 1.0 to 5.0 µm, preferably 1.5 to 4.5 µm, preferably 2.0 to 3.5 µm.

7. A method as claimed in any of claims 1 to 3 or 5 to 6 or a medicament as claimed in claim 4, wherein the particles of one or more further particulate ingredients have an aerodynamic particle size of 1.0 to 5.0 µm, preferably 1.5 to 4.5 µm, preferably 2.0 to 3.5 µm.

8. An inhalable dry-powder medicament obtainable by the method as claimed in any of claims 1 to 3 or 5 to 7.

## Patentansprüche

1. Verfahren zur Herstellung eines inhalierbaren Trockenpulvermedikaments, umfassend die folgenden Schritte:
(i) Fraktionieren eines partikelförmigen Wirkstoffs auf Grundlage der aerodynamischen Partikelgröße,
(ii) Rückgewinnung von mindestens einer Fraktion des partikelförmigen Wirkstoffs und
(iii) Kombinieren der zurückgewonnenen Fraktion mit einem Trägerstoff, um das inhalierbare Trockenpulvermedikament bereitzustellen;
ferner umfassend die Schritte der Fraktionierung von einem oder mehreren weiteren partikelförmigen Inhaltsstoffen auf Grundlage der aerodynamischen Partikelgröße, der Rückgewinnung von mindestens einer Fraktion der einen oder mehreren weiteren partikelförmigen Inhaltsstoffe und der Kombination der zurückgewonnenen Fraktion(en) mit dem inhalierbaren Trockenpulvermedikament;
wobei die einen oder mehreren weiteren partikelförmigen Inhaltsstoffe einen weiteren partikelförmigen Wirkstoff und einen partikelförmigen feinteiligen Trägerstoff umfassen;
wobei der partikelförmige feinteilige Trägerstoff Lactose mit feinen Partikeln ist; und wobei der partikelförmige Wirkstoff und der weitere partikelförmige Wirkstoff Fluticasonpropionat und Salmeterolxinafoat umfassen.

2. Verfahren nach Anspruch 1, wobei die Fraktionierung unter Verwendung eines NGI durchgeführt wird.

3. Verfahren nach Anspruch 2, wobei die zurückgewonnene Fraktion aus einem Stadium oder Stadien mit einer oberen Ausschlussgrenze von 7,0-9,0 µm und einer unteren Ausschlussgrenze von 2,5-3,0 µm stammt, bestimmt bei einer Durchflussrate von 60±5 L/min.

4. Inhalierbares Trockenpulvermedikament, umfassend einen partikelförmigen Wirkstoff und einen Trägerstoff, wobei der partikelförmige Wirkstoff auf Grundlage der aerodynamischen Partikelgröße vorfraktioniert wird;
ferner umfassend einen oder mehrere weitere partikelförmige Inhaltsstoffe, die auf Grundlage der aerodynamischen Partikelgröße vorfraktioniert werden;
wobei die einen oder mehreren weiteren partikelförmigen Inhaltsstoffe einen weiteren partikelförmigen Wirkstoff und einen partikelförmigen feinteiligen Trägerstoff umfassen;
wobei der partikelförmige feinteilige Trägerstoff Lactose mit feinen Partikeln ist; und
wobei der partikelförmige Wirkstoff und der weitere partikelförmige Wirkstoff Fluticasonpropionat und Salmeterolxinafoat umfassen.

5. Verfahren nach einem der vorhergehenden Ansprüche, wobei der Trägerstoff Lactose mit groben Partikeln ist.

6. Verfahren nach einem der vorhergehenden Ansprüche, wobei die Partikel in der zurückgewonnenen Fraktion des partikelförmigen Wirkstoffs eine aerodynamische Partikelgröße von 1,0 bis 5,0 µm, vorzugsweise 1,5 bis 4,5 µm, vorzugsweise 2,0 bis 3,5 µm aufweisen.

7. Verfahren nach einem der Ansprüche 1 bis 3 oder 5 bis 6 oder Medikament nach Anspruch 4, wobei die Partikel von einem oder mehreren weiteren partikelförmigen Inhaltsstoffen eine aerodynamische Partikelgröße von 1,0 bis 5,0 µm, vorzugsweise 1,5 bis 4,5 µm, vorzugsweise 2,0 bis 3,5 µm aufweisen.

8. Inhalierbares Trockenpulvermedikament, das durch das Verfahren nach einem der Ansprüche 1 bis 3 oder 5 bis 7 erhalten werden kann.

## Revendications

1. Procédé de préparation d'un médicament sous forme de poudre sèche inhalable comprenant les étapes suivantes :
(i) fractionnement d'un ingrédient actif particulaire en fonction de la taille des particules aérodynamiques,
(ii) récupération d'au moins une fraction de l'ingrédient actif particulaire et
(iii) combinaison de la fraction récupérée avec un véhicule permettant d'administrer le médicament sous forme de poudre sèche inhalable;
comprenant en outre les étapes de fractionnement d'un ou plusieurs autres ingrédients particulaires en fonction de la taille des particules aérodynamiques, récupération d'au moins une partie d'un ou plusieurs autres ingrédients particulaires et combinaison de la (des) fraction(s) récupérée(s) avec le médicament sous forme de poudre sèche inhalable;
où l'un ou plusieurs autres ingrédients particulaires comprennent un autre ingrédient actif particulaire et un véhicule de fines particules;
où le véhicule de fines particules est du lactose en fines particules; et
où l'ingrédient actif particulaire et un autre ingrédient actif particulaire comprennent le propionate de fluticasone et le xinafoate de salmétérol.

2. Procédé selon la revendication 1, où le fractionnement est réalisé à l'aide d'un NGI.

3. Procédé selon la revendication 2, où la fraction récupérée provient d'une étape ou de plusieurs étapes dans lesquelles la limite de sélection supérieure est de 7,0-9,0 µm la limite de sélection inférieure est de 2,5-3,0 µm déterminée pour un débit de 60 ± 5 L/min.

4. Médicament sous forme de poudre sèche inhalable comprenant un ingrédient actif particulaire et un véhicule, où l'ingrédient actif particulaire est préfractionné en fonction de la taille des particules aérodynamiques;
comprenant en outre un ou plusieurs autres ingrédients particulaires qui sont préfractionnés en fonction de la taille des particules aérodynamiques;
où l'un ou plusieurs autres ingrédients particulaires comprennent un autre ingrédient actif particulaire et un véhicule de fines particules;
où le véhicule de fines particules est du lactose en fines particules; et
où l'ingrédient actif particulaire et un autre ingrédient actif particulaire comprennent le propionate de fluticasone et le xinafoate de salmétérol.

5. Procédé selon l'une quelconque des revendications précédentes, où le véhicule est du lactose en particules grossières.

6. Procédé selon l'une quelconque des revendications précédentes, où les particules dans la fraction récupérée de l'ingrédient actif particulaire ont une taille de particules aérodynamiques de 1,0 à 5,0 µm, de préférence de 1,5 à 4,5 µm, de préférence de 2,0 à 3,5 µm.

7. Procédé selon l'une quelconque des revendications 1 à 3 ou 5 à 6 ou un médicament selon la revendication 4, où les particules d'un ou plusieurs autres ingrédients particulaires ont une taille de particules aérodynamiques de 1,0 à 5,0 µm, de préférence de 1,5 à 4,5 µm, de préférence de 2,0 à 3,5 µm.

8. Médicament sous forme de poudre sèche inhalable pouvant être obtenu grâce au procédé décrit dans l'une quelconque des revendications 1 à 3 ou 5 à 7.
